# EUROPEAN PATENT APPLICATION

(11) **EP 4 119 656 A1**
(43) Date of publication of application: **18.01.2023**
(21) Application number: 21767695.6
(22) Date of filing: 12.03.2021
(51) Int. Cl.: C12N 5/071, A61L 27/36, A61L 27/38, C07K 14/705, C12Q 1/04, G01N 15/14, G01N 33/53

(54) **CELL CULTURE, METHOD FOR EVALUATING CELL CULTURE, METHOD FOR PRODUCING CELL CULTURE, AND MARKER FOR USE IN EVALUATION OF CHONDROID TISSUE FORMATION PROPERTY**

(30) Priority: 13.03.2020 JP 2020044265
(71) Applicant: Tokai University Educational System, Tokyo 1518677 (JP); CellSeed Inc., Tokyo 135-0064 (JP)
(72) Inventor: SATO Masato, Isehara-shi, Kanagawa 259-1193 (JP); TAKAHASHI Takumi, Isehara-shi, Kanagawa 259-1193 (JP); KAWAGUCHI Yuka, Tokyo 135-0064 (JP); SATO Chikako, Tokyo 135-0064 (JP); TSUNODA Satoshi, Tokyo 135-0064 (JP); TOHYAMA Chiharu, Tokyo 135-0064 (JP); SONG Dandan, Tokyo 135-0064 (JP); MIYAZAWA Michihide, Tokyo 135-0064 (JP)
(74) Representative: Godemeyer Blum Lenze Patentanwälte Partnerschaft mbB - werkpatent
(86) International application number: PCT/JP2021/010208
(87) International publication number: WO 2021/182633

(57) **Abstract**

[Problems] To provide a cell culture which is suitable for use for cartilage repair.

[Solution] The present invention provides a cell culture having a cartilage-like tissue forming property, comprising a cell population in which the expression intensity of at least one cell surface marker selected from the group consisting of CD166, CD165, CD99, GD2, STRO-1, CD108, CD164, CD6, CD106, and CD107b is not higher than the threshold for each cell surface marker, and/or, the expression intensity of at least one cell surface marker selected from the group consisting of CD26, CD73, CD105, CD44, CD120a, CD201, EGFR, CD146, CD140a, and CD90 is not lower than the threshold for each cell surface marker.

## Description

### Technical Field

The present technology relates to a cell culture, a method for evaluating a cell culture, a method for producing a cell culture, and a marker for evaluating a cartilage-like tissue forming property. More specifically, the present technology relates to a cell culture containing a cell population expressing a specific cell surface marker, a method for evaluating a cell culture based on a specific cell surface marker, a method for producing a cell culture containing a cell population expressing a specific cell surface marker, and a specific cell surface marker used for evaluating a cartilage-like tissue forming property.

### Background Art

For the treatment of locomotory diseases such as osteoarthritis, a treatment of cartilage tissue using the tissue regeneration engineering technology is performed. In this treatment, cultured chondrocytes or chondrocyte-based cartilage tissue can be transplanted to the affected area. Various transplantation materials have been proposed so far.

For example, Patent Literature 1 below describes "A transplantation material to be transplanted to a predetermined transplantation site, which is an antigenic suppression treatment while maintaining the shape of the tissue structure in a tissue structure of the same kind as the predetermined transplantation site obtained from body tissue. A material for transplantation in which cells corresponding to the predetermined transplantation site are retained in the cell-retaining carrier obtained by subjecting." (claim 1).

### Citation List

### Patent Literature

Patent Literature 1: Japanese Unexamined Patent Application Publication (JP-A) No. 2003-180819

### Summary of Invention

### Technical Problem

A main object of the present invention is to provide a cell culture suitable for cartilage repair, particularly a cell culture suitable for hyaline cartilage repair.

### Solution to Problem

The inventors have found that a cell culture with particular characteristics is suitable for cartilage repair, especially hyaline cartilage repair.

That is, the present invention provides a cell culture having a cartilage-like tissue forming property, comprising a cell population in which the expression intensity of at least one cell surface marker selected from the group consisting of CD166, CD165, CD99, GD2, STRO-1, CD108, CD164, CD6, CD106, and CD107b is not higher than the threshold for each cell surface marker, and/or, the expression intensity of at least one cell surface marker selected from the group consisting of CD26, CD73, CD105, CD44, CD120a, CD201, EGFR, CD146, CD140a, and CD90 is not lower than the threshold for each cell surface marker.

The present invention also provides a method for evaluating a cell culture, comprising a determination step of determining whether the expression intensity of at least one cell surface marker selected from the group consisting of CD166, CD165, CD99, GD2, STRO-1, CD108, CD164, CD6, CD106, and CD107b is not higher than the threshold for each surface marker, and/or, the expression intensity of at least one cell surface marker selected from the group consisting of CD26, CD73, CD105, CD44, CD120a, CD201, EGFR, CD146, CD140a, and CD90 is not lower than the threshold for each surface marker, for the cell population contained in the cell culture.

In addition, the present invention also provides a method for producing a cell culture having a cartilage-like tissue forming property, comprising a culturing step of culturing cells to obtain a cell culture, wherein the culturing is performed so that the expression intensity of at least one cell surface marker selected from the group consisting of CD166, CD165, CD99, GD2, STRO-1, CD108, CD164, CD6, CD106, and CD107b is not higher than the threshold for each cell surface marker, and/or, the expression intensity of at least one cell surface marker selected from the group consisting of CD26, CD73, CD105, CD44, CD120a, CD201, EGFR, CD146, CD140a, and CD90 is not lower than the threshold for each cell surface marker, regarding the cell population contained in the cell culture, in the culturing step.

In addition, the present invention also provides a marker for evaluating a cartilage-like tissue forming property, comprising at least one cell surface marker selected from the group consisting of CD166, CD165, CD99, GD2, STRO-1, CD108, CD 164, CD6, CD 106, and CD107b, and/or, at least one cell surface marker selected from the group consisting of CD26, CD73, CD105, CD44, CD120a, CD201, EGFR, CD146, CD140a, and CD90.

### Advantageous Effects of Invention

The present invention provides a cell culture suitable for use in cartilage repair, particularly hyaline cartilage repair. Further, the present invention also makes it possible to evaluate the ability of a cell culture to repair cartilage, particularly the ability to repair hyaline cartilage.

### Brief Description of Drawings

[Fig. 1] A flow diagram of an experiment.
[Fig. 2] A graph showing the gene expression ratio between COL2A1 and COL1A1.
[Fig. 3] A diagram showing staining results.

### Description of Embodiments

### 1. Cell culture

The present invention provides a cell culture having a cartilage-like tissue forming property, more particularly a cell culture having a hyaline cartilage-like tissue forming property. In the cell population contained in the cell culture, the expression intensity of at least one cell surface marker selected from the group consisting of CD166, CD165, CD99, GD2, STRO-1, CD108, CD164, CD6, CD106, and CD107b may be not higher than the threshold for each cell surface maker, and/or, the expression intensity of at least one cell surface marker selected from the group consisting of CD26, CD73, CD105, CD44, CD120a, CD201, EGFR, CD146, CD140a, and CD90 may be not lower than the threshold for each cell surface marker. By having these cell surface markers not lower than or not higher than a predetermined threshold, the above-described cell culture is suitable for cartilage repair, more particularly for knee cartilage repair.

The cell culture of the present invention has a property of forming a cartilage-like tissue, particularly a hyaline cartilage-like tissue. The cell culture of the present invention has a property of forming a cartilage-like tissue, particularly a hyaline cartilage-like tissue, when transplanted to a human body, more particularly to a knee joint cartilage portion of a human body. As used herein, the term "cartilage-like tissue" may be a tissue having components and/or functions equivalent to or similar to those of cartilage tissue (especially knee joint cartilage tissue) and, for example, refers to a tissue expressing type 2 collagen like hyaline cartilage tissue.

In the present invention, it is preferable that the expression intensity of at least CD99 and/or GD2 among the cell surface markers is not higher than the threshold for each of these cell surface markers. When the expression intensity of CD99 and/or GD2 is not higher than a predetermined threshold, the cell culture can exhibit a particularly good cartilage-like tissue forming property, especially a hyaline cartilage-like tissue forming property.

In the present invention, it is preferable that the expression intensity of at least CD26 and/or CD73 among the cell surface markers is not lower than the threshold for each of these cell surface markers. When the expression intensity of CD26 and/or CD73 is not lower than a predetermined threshold, the cell culture can exhibit a particularly good cartilage-like tissue forming property, particularly a hyaline cartilage-like tissue forming property.

It is particularly preferable that the expression intensity of at least CD99 and/or GD2 among the cell surface markers is not higher than the threshold for each of these cell surface markers, and the expression intensity of at least CD26 and/or CD73 among the cell surface markers is not lower than the threshold for each of these cell surface markers. If these four cell surfaces meet the above-described criteria for the threshold, the cell culture can exhibit a particularly good cartilage-like tissue forming property, in particular a hyaline cartilage-like tissue forming property.

Further, in the present invention, it is preferable that the expression intensity of any one, two, or three of at least CD26, CD73, and CD44 among the cell surface markers is not lower than the threshold for each of these cell surface markers. When the expression intensity of any one, two, or three of CD26, CD73, and CD44 is not lower than a predetermined threshold, the cell culture can exhibit a particularly good cartilage-like tissue forming property, particularly a hyaline cartilage-like tissue forming property.

It is particularly preferable that the expression intensity of at least CD99 and/or GD2 among the cell surface markers is not higher than the threshold for each of these cell surface markers, and the expression intensity of any one, two, or three of at least CD26, CD73, and CD44 among the cell surface markers is not lower than the threshold for each of these cell surface markers. If these four cell surfaces meet the above-described criteria for the threshold, the cell culture can exhibit a particularly good cartilage-like tissue forming property, in particular a hyaline cartilage-like tissue forming property.

In one preferred embodiment of the present invention, the expression intensity of at least CD44 among the cell surface markers is not lower than the threshold for this cell surface marker. When the expression intensity of CD44 is not lower than a predetermined threshold, the cell culture can exhibit a particularly good cartilage-like tissue forming property, particularly a hyaline cartilage-like tissue forming property.

It is particularly preferable that the expression intensity of at least CD99 and/or GD2 among the cell surface markers is not higher than the threshold for each of these cell surface markers, and the expression intensity of at least CD44 among the cell surface markers is not lower than the threshold for this cell surface marker. If these three cell surfaces meet the above-described criteria for the threshold, the cell culture can exhibit a particularly good cartilage-like tissue forming property, in particular a hyaline cartilage-like tissue forming property.

In the present invention, the expression intensity of a cell surface marker may be the normalized mean fluorescence intensity (nMFI, normalized Mean Fluorescence Intensity) measured by analyzing a cell population by flow cytometry. The normalized mean fluorescence intensity is suitable for evaluation of a cartilage-like tissue forming property, particularly a hyaline cartilage-like tissue forming property, based on these cell surface markers. The normalized mean fluorescence intensity may be measured by the measurement method described in "5. Example" below.

In the cell culture of the present invention, it is preferable that the normalized mean fluorescence intensity measured when analyzing the cell population contained in the cell culture by flow cytometry satisfies at least one of the following conditions 1 to 10, and/or, satisfies at least one of the following conditions 11 to 20.
Condition 1: when the cell population is labeled with a PE-bound anti-CD166 antibody, the normalized mean fluorescence intensity derived from the PE is 240 or less, more preferably 220 or less, and further more preferably 200 or less,
Condition 2: when the cell population is labeled with a BB700-bound anti-CD165 antibody, the normalized mean fluorescence intensity derived from the BB700 is 132 or less, more preferably 120 or less, and further more preferably 110 or less,
Condition 3: when the cell population is labeled with an FITC-bound anti-CD99 antibody, the normalized mean fluorescence intensity derived from the FITC is 1.9 or less, more preferably 1.8 or less, and further more preferably 1.6 or less,
Condition 4: when the cell population is labeled with a BB700-bound anti-GD2 antibody, the normalized mean fluorescence intensity derived from the BB700 is 3.6 or less, more preferably 3.3 or less, and further more preferably 3.0 or less,
Condition 5: when the cell population is labeled with an FITC-bound anti-STRO-1 antibody, the normalized mean fluorescence intensity derived from the FITC is 1.4 or less, more preferably 1.3 or less, and further more preferably 1.2 or less,
Condition 6: when the cell population is labeled with a PE-bound anti-CD108 antibody, the normalized mean fluorescence intensity derived from the PE is 1.9 or less, more preferably 1.8 or less, and further more preferably 1.6 or less,
Condition 7: when the cell population is labeled with a PE-bound anti-CD164 antibody, the normalized mean fluorescence intensity derived from the PE is 3.0 or less, more preferably 2.8 or less, and further more preferably 2.5 or less,
Condition 8: when the cell population is labeled with an FITC-bound anti-CD6 antibody, the normalized mean fluorescence intensity derived from the FITC is 1.4 or less, more preferably 1.3 or less, and further more preferably 1.2 or less,
Condition 9: when the cell population is labeled with an FITC-bound anti-CD106 antibody, the normalized mean fluorescence intensity derived from the FITC is 2.4 or less, more preferably 2.2 or less, and further more preferably 2.0 or less,
Condition 10: when the cell population is labeled with an FITC-bound anti-CD107b antibody, the normalized mean fluorescence intensity derived from the FITC is 2.0 or less, more preferably 1.9 or less, and further more preferably 1.7 or less,
Condition 11: when the cell population is labeled with an FITC-bound anti-CD26 antibody, the normalized mean fluorescence intensity derived from the FITC is 2.3 or more, more preferably 2.6 or more, and further more preferably 2.9 or more,
Condition 12: when the cell population is labeled with an FITC-bound anti-CD73 antibody, the normalized mean fluorescence intensity derived from the FITC is 47 or more, more preferably 53 or more, and further more preferably 59 or more,
Condition 13: when the cell population is labeled with a PE-bound anti-CD105 antibody, the normalized mean fluorescence intensity derived from the PE is 21 or more, more preferably 24 or more, and further more preferably 27 or more,
Condition 14: when the cell population is labeled with an FITC-bound anti-CD44 antibody, the normalized mean fluorescence intensity derived from the FITC is 128 or more, more preferably 145 or more, and further more preferably 160 or more,
Condition 15: when the cell population is labeled with an APC-bound anti-CD120a antibody, the normalized mean fluorescence intensity derived from the APC is 24 or more, more preferably 27 or more, and further more preferably 30 or more,
Condition 16: when the cell population is labeled with a PE-bound anti-CD201 antibody, the normalized mean fluorescence intensity derived from the PE is 18 or more, more preferably 20 or more, and further more preferably 23 or more,
Condition 17: when the cell population is labeled with a PE-bound anti-EGFR antibody, the normalized mean fluorescence intensity derived from the PE is 3.2 or more, more preferably 3.6 or more, and further more preferably 4.0 or more,
Condition 18: when the cell population is labeled with an FITC-bound anti-CD146 antibody, the normalized mean fluorescence intensity derived from the FITC is 1.6 or more, more preferably 1.8 or more, and further more preferably 2.0 or more,
Condition 19: when the cell population is labeled with a PE-bound anti-CD140a antibody, the normalized mean fluorescence intensity derived from the PE is 5.6 or more, more preferably 6.4 or more, and further more preferably 7.0 or more,
Condition 20: when the cell population is labeled with an APC-bound anti-CD90 antibody, the normalized mean fluorescence intensity derived from the APC is 800 or more, more preferably 900 or more, and further more preferably 1000 or more.

By satisfying any one or more of these conditions, the cell culture of the present invention has a more favorable cartilage-like tissue forming property, particularly a hyaline cartilage-like tissue forming property.

It is particularly preferable that the normalized mean fluorescence intensity measured when analyzing the cell population by flow cytometry satisfies the conditions 3 and/or 4, and/or, satisfied the conditions 11 and/or 12. It is even more preferable that the normalized mean fluorescence intensity measured when analyzing the cell population by flow cytometry satisfies the conditions 3 and 4, and, satisfies the conditions 11 and/or 12. The fulfillment of one, two, three or all four of these four conditions, in particular the fulfillment of all four, gives the cell culture of the present invention a particularly good cartilage-like tissue forming property, in particular a hyaline cartilage-like tissue forming property.

In one embodiment of the present invention, the normalized mean fluorescence intensity measured when analyzing the cell population by flow cytometry satisfies the conditions 3 and/or 4, and/or, satisfies one, two, or three of the conditions 11, 12 and 14. It is even more preferable that the normalized mean fluorescence intensity measured when analyzing the cell population by flow cytometry satisfies the conditions 3 and 4, and satisfies the conditions 11, 12 and 14. The fulfillment of one, two, three, four or all five of these five conditions, in particular the fulfillment of all five, gives the cell culture of the present invention a particularly good cartilage-like tissue forming property, in particular a hyaline cartilage-like tissue forming property.

In one embodiment of the present invention, the normalized mean fluorescence intensity measured when analyzing the cell population by flow cytometry satisfies the conditions 3 and/or 4, and/or, satisfies the condition 14. It is even more preferable that the normalized mean fluorescence intensity measured when analyzing the cell population by flow cytometry satisfies the conditions 3 and 4, and, satisfies the condition 14. The fulfillment of one, two or all three of these three conditions, gives the cell culture of the present invention a particularly good cartilage-like tissue forming property, in particular a hyaline cartilage-like tissue forming property.

### (Shape of cell culture)

According to one embodiment of the present invention, the above-described cell culture may be in solid form. For example, the cell population contained in the cell culture may be organized into any shape. The shape of the solid cell culture may be, for example, in a sheet form, granular, fibrous (thread form), or in a net form (mesh form).

According to another embodiment of the present invention, the above-described cell culture may not be solid, and for example, it may be flowable. The non-solid cell culture may be, for example, fluid, liquid, or sol.

The cell culture of the present invention is preferably in a sheet form. The cell culture in a sheet form is particularly suitable for cartilage repair, especially hyaline cartilage repair. When the cell culture is in a sheet form, the thickness of the sheet-form cell culture may be, for example, 4 µm to 5 mm, particularly 4 µm to 3 mm, and more particularly 4 µm to 1 mm. The sheet-like cell culture may be a single sheet of the sheet-form cell culture, or may be a laminate composed of a multiple sheet (for example, 2 to 10 sheets, particularly 2 to 8 sheets, more particularly 2 to 5 sheets) of the sheet-form cell culture.

More particularly, the thickness of the sheet-form cell culture may be, for example, 4 µm to 100 µm, preferably 4 µm to 70 µm, and more preferably 4 µm to 50 µm.

The sheet-form cell culture of the present invention may be one in which cells are naturally stratified during the cell growth process. The sheet-form cell culture of the present invention does not have to be one in which two or more separately produced sheet-form cell cultures are artificially layered and cultured continuously.

### (Cell culture derived from cartilage tissue)

In one embodiment of the present invention, the cell culture may be derived from cartilage tissue. That is, the cell culture may be a culture of cartilage tissue-derived cells, and in particular, the raw material cells used for culturing to obtain the cell culture may be cartilage tissue-derived cells. The cell culture is not a natural product because it is obtained by artificial culture outside a living body. The cartilage tissue-derived cells may be, for example, a plurality of cells obtained by separating cells contained in cartilage tissue from the cartilage substrate. For example, the cartilage tissue-derived cells may be a plurality of cells obtained by treating the cartilage tissue with an enzyme to release the cells in the cartilage tissue from the cartilage substrate, and then recovering the released cells by centrifugation.

The above-described cell culture is preferably not derived from the synovium. That is, the cell culture preferably does not contain synovium-derived cells. The above-described cell culture is preferably not formed from a culture of synovium-derived cells, nor is it formed from a culture of synovial cells. The cell culture of the present invention may preferably be a culture of only cartilage tissue-derived cells, particularly a culture of only hyaline cartilage tissue-derived cells.

In the present invention, the cartilage tissue-derived cells may be derived from the cartilage tissue of a polydactyly animal, or may be derived from the cartilage tissue of a polymelia animal. The animal may preferably be a mammal, more preferably a primate, and further more preferably a human. The cartilage tissue may be harvested from tissue obtained, for example, during surplus finger excision. The tissue may be, for example, a portion that does not appear white when photographed with an X-ray, that is, a portion that appears as a black void. Polydactyly may be of the distal, middle, or proximal phalanx type. The surplus finger may be any finger, for example the thumb or little finger. If the surplus finger (limb) to be collected is warty and small, all the collected subcutaneous tissue can be used. When the animal is human, the age of the human is not limited, but can be, for example, 5 years old or younger, 3 years old or younger, or 2 years old or younger.

Examples of enzymes used in the enzymatic treatment include collagenase, caseinase, clostripain, trypsin, hyaluronidase, elastase, pronase, and dispase. Preferably, combinations of these enzymes may be used. Examples of preferred enzyme combinations are, for example, combinations of collagenase, caseinase, clostripain, and trypsin. Enzyme preparations containing this combination include, but not limited to, for example, collagenase type I, collagenase type II, collagenase type III, collagenase type IV, and collagenase type V (all available from FUJIFILM Wako Pure Chemical Corporation). Further, other examples of preferred enzyme combinations are, for example, combinations of collagenase and dispase or thermolysin. Enzyme preparations containing this combination include, but are not limited to, for example, Liberase (available from Roche Diagnostics K.K.). In addition, depending on the state of the tissue, the enzyme treatment may be performed stepwise with a plurality of types of enzymes. For example, isolation may be performed by treatment with collagenase, caseinase, clostripain and trypsin in that order. Enzymatic treatment conditions can be appropriately determined by those skilled in the art depending on the type of enzyme used and/or the state of cartilage tissue. The enzymatic treatment may be carried out, for example, at 30 to 50°C, preferably 33 to 45°C, or 35 to 40°C, for 1 to 12 hours, preferably 2 to 5 hours. If the enzyme treatment temperature is too high, problems such as cell denaturation, reduction in viable cells, reduction in proliferation ability, and inability to isolate may occur. In addition, when the enzyme treatment temperature is too low, sufficient enzyme activity may not be achieved and cells may not be isolated in some cases. During the enzymatic treatment, cells can be collected with high efficiency by applying a physical stimulus.

The enzymatic reaction may be stopped by washing and diluting the cell suspension containing enzymatically treated articular cartilage. After stopping the enzymatic reaction, the cell suspension may be separated into the cell mass and the supernatant by centrifugation. For Liberase, two or more washes may stop the enzymatic reaction. The centrifugation may be performed under conditions that allow more cells with a size of less than 25 µm, particularly 20 µm or less, and 15 µm or more to be collected. In order to collect more of such cells, the centrifugation may be performed, for example, at 1000 rpm or more, 1500 rpm or more, or 2000 rpm or more, for example, for 5 minutes or more, 7 minutes or more, or 10 minutes or more.

The cartilage tissue-derived cells may be obtained by a so-called outgrowth method. The outgrowth method can include the steps of cutting the collected cartilage tissue into small pieces, seeding the minced pieces of cartilage tissue into a culture dish with a small amount of culture medium, and culturing the pieces. Proliferated cells are produced from the cartilage tissue piece by the culture. The generated cells are harvested by enzymatic treatment and centrifugation. The collected cells may be used for producing the cell sheet of the present invention.

The step of mincing the cartilage tissue may be performed, for example, in a wet state. This step may be carried out, for example, by placing tissue pieces and a small amount of medium in a 50 ml centrifuge tube and chopping them with Metzenbaum Scissors, SuperCut Tungsten Carbide 18 cm Long Curve (manufactured by World Precision Instruments). It is preferable to obtain the smallest possible cartilage tissue pieces. Further, the medium for culturing the minced cartilage tissue piece can be appropriately selected by those skilled in the art, and may be preferably DMEM/F12 + 20% FBS + antibiotics (hereinafter also referred to as AB). After confirmation of cell adhesion to the culture dish after initiation of the culture, the medium may be preferably replaced with DMEM/F12 + 20% FBS + AB + ascorbic acid (hereinafter also referred to as AA). If the medium contains ascorbic acid from the beginning of the culture, cell adhesion to the culture dish can be inhibited. Further, the culture may be performed under general culture conditions, for example, in an incubator at 37°C and 5% CO₂. The culturing may be performed until subconfluence is achieved. The enzyme preparation used in harvesting cells produced in culture can also contain, for example, trypsin and EDTA. The centrifugation may be performed as described above.

In the present invention, the cartilage tissue-derived cells may preferably contain mesenchymal stem cells. The cartilage tissue-derived cells may further include cells contained in cartilage tissue in addition to mesenchymal stem cells. That is, in the present invention, the cartilage tissue-derived cells can be a population of multiple types of cells including mesenchymal stem cells. Examples of cells other than mesenchymal stem cells include, but are not limited to, chondrocytes and chondroblasts. The cell culture of the present invention is more suitable for cartilage repair because it is formed from a culture of the cartilage tissue-derived cells.

### (Cell culture derived from stem cells)

In another embodiment of the present invention, the cell culture may be derived from stem cells. The stem cells may include pluripotent stem cells, embryonic stem cells, or somatic stem cells, and may include, for example, iPS cells.

In this embodiment, for example, pluripotent stem cells (particularly iPS cells) are differentiated by culturing in a medium to obtain chondrocytes or chondrocyte-like cells, and the chondrocytes or chondrocyte-like cells are further cultured, for example, on the surface on which a stimulus-responsive polymer is immobilized, thus, the cell culture of the present invention is obtained. For example, the cell culture of the present invention can also be obtained by seeding pluripotent stem cells (especially iPS cells) on a cultureware on which a stimulus-responsive polymer is immobilized, and culturing the cells after differentiation into chondrocytes or chondrocyte-like cells.

### (Application of cell culture)

The cell culture of the present invention may be used for repairing cartilage tissue, more preferably for repairing knee cartilage tissue, and further more preferably for repairing knee hyaline cartilage tissue. The cell culture of the present invention is suitable for the above-described repair because it has a cartilage-like tissue forming property, in particular a hyaline cartilage-like tissue forming property.

In the present invention, the repair of cartilage tissue includes, but not limited to, treating cartilage tissue having inflammation and/or damage, reinforcing cartilage tissue, supplementing defective portions of cartilage tissue, and regenerating cartilage tissue. Also, the cell culture of the present invention may be used to prevent diseases related to cartilage tissue. The cell culture of the present invention can be applied, for example, to diseased cartilage or bone tissue. Examples of diseases to which the cell culture of the present invention is applied include, but are not limited to, arthritis, arthrosis, cartilage injury, osteo-cartilaginous injury, meniscus injury, and/or intervertebral disc degeneration.

The cell culture of the present invention is more particularly suitable for surgical treatment, particularly surgical therapy of cartilage tissue. The cartilage repair using the cell culture of the present invention can be performed, for example, by surgically exposing a cartilage portion in need of repair and applying the cell culture to the exposed portion.

For example, a sheet-form cell culture of the present invention may be applied to the exposed portion. The number and size of the sheet-form cell culture to be administered can be appropriately determined by those skilled in the art, taking into account, for example, the condition of the area to be treated or the type of the disease. In addition, in the sheet-form cell culture of the present invention, the subchondral bone can preferably be treated so that bleeding from the subchondral bone is confirmed before the sheet-form cell culture is applied. The above-described treatment may be carried out by methods known to the person skilled in the art, for example by microfracture or drilling. This treatment further promotes cartilage repair by the sheet-form cell culture of the present invention.

When applying the sheet-form cell culture of the present invention to the affected area, bonding or suturing with an adhesive that can be used in vivo may be performed. Alternatively, the sheet-form cell culture may simply be adhered to the affected area without performing the bonding or suturing.

When the cell culture of the present invention is a sheet-form cell culture, the sheet-form cell culture may have substrates on all surfaces. Preferably, the above-described substrate may comprise fibronectin. When cells are cultured on a cultureware, substrates are usually produced between the cells and the cultureware, and substrates are not produced on the opposite side of the cultureware, that is, the portion not in contact with the cultureware. The sheet-form cell culture of the present invention can have substrates produced not only on the surface in contact with the cultureware but also on the surface not in contact with the cultureware. The sheet-form cell culture of the present invention can be made more suitable for cartilage repair by having substrates on all surfaces of the sheet-form cell culture.

Preferably, in the sheet-form cell culture of the present invention, basement membrane-like proteins formed between cells and porous membranes during culture can be those not destroyed by enzymes such as proteases such as dispase and trypsin. That is, the sheet-form cell culture of the present invention can have basement membrane-like proteins between cells and porous membranes. In particular, the sheet-form cell culture of the present invention can have basement membrane-like proteins between cells and porous membranes on one surface of the sheet-form cell culture or on both surfaces of the sheet-form cell culture. By having the basement membrane-like protein in the sheet-form cell culture, the sheet-form cell culture can exhibit better cartilage repair ability.

The sheet-form cell culture of the present invention has preferably a cell density of 100×10⁵ to 100×10⁸ cells/cm³, more preferably 100×10⁶ to 100×10⁷ cells/cm³, more preferably 100×10⁶ to 500×10^{6/}cm³, and further more preferably 200×10⁶ to 300×10^{6/}cm³.

Preferably, the cell culture of the present invention does not contain artificial scaffold components. That is, the cell culture of the present invention may consist only of cells in the culture and components produced from the cells (and medium components attached to the cell sheet). The cell culture of the present invention may be preferably transplanted to patients without containing artificial scaffolding components.

The cell culture of the present invention can be produced according to the production method of the present invention described in 2. below. Therefore, please see 2. below, for the method for producing the cell culture of the present invention.

### 2. Method for producing cell culture

The present invention also provides a method for producing a cell culture with a cartilage-like tissue forming property. The production method includes a culturing step of culturing cells to obtain a cell culture. The production method may further comprise an analysis step of analyzing the expression intensity of a cell surface marker with respect to the cell population contained in the cell culture, and a determining step of determining whether the cell culture has a cartilage-like tissue forming property based on the expression intensity obtained in the analysis step. Each step will be described below.

### 2-1. Culturing step

The culturing may be performed so that the expression intensity of at least one cell surface marker selected from the group consisting of CD166, CD165, CD99, GD2, STRO-1, CD108, CD164, CD6, CD106, and CD107b is not higher than the threshold for each cell surface marker, and/or, the expression intensity of at least one cell surface marker selected from the group consisting of CD26, CD73, CD105, CD44, CD120a, CD201, EGFR, CD146, CD140a, and CD90 is not lower than the threshold for each cell surface marker, regarding the cell population contained in the cell culture, in the culturing step.

The cell culture obtained by the culturing step has an excellent cartilage-like tissue forming property, particularly a hyaline cartilage-like tissue forming property. The cell culture can form cartilage-like tissue, particularly hyaline cartilage-like tissue, for example, when transplanted into a human body, particularly into a knee cartilage portion in a human body. That is, the cell culture can exhibit cartilage repairing activity, particularly hyaline cartilage repairing activity. Then, the cell culture exhibits functions similar to those of cartilage tissue.

The cells cultured in the culturing step may be the cartilage tissue-derived cells described in 1. above, or may be stem cell-derived cells. For example, the raw material cells used for culture may be the cartilage tissue-derived cells, and the cartilage tissue-derived cells may be cells obtained by culturing cells in cartilage tissue for at least two days in DMEM/F12 containing FBS.

In a preferred embodiment of the present invention, the raw material cells used for culturing to obtain the cell culture may be those prepared by a raw material cell preparation method comprising a first culturing step of culturing the cells in the cartilage tissue in a medium to make it confluent, a dissociation step of dissociating the cell population from each other made confluent in the first culturing step, and a second culturing step of further culturing the cell population dissociated in the dissociation step in the same fresh medium as the above-described medium. The raw material cells prepared by this raw material cell preparation method are suitable for production of tissue cultures having suitability for cartilage repair.

In the culturing step, the cells may be preferably cultured in a medium containing a cultureware having a surface on which a stimulus-responsive polymer is immobilized, and for example, the cells may be cultured on the porous membrane surface on which a stimulus-responsive polymer is immobilized. By culturing cells in a medium containing a cultureware (in particular, a porous membrane) having a surface on which a stimulus-responsive polymer is immobilized, a cell culture having a cartilage-like tissue forming property can be obtained, and it may also be peeled off from the cultureware without damaging the cell culture. The above-described stimulus-responsive polymer may be, for example, a temperature-responsive polymer, a pH-responsive polymer, or a photo-responsive polymer, and more specifically may be a polymer that changes properties (e.g., hydration force) by temperature stimulus (e.g., temperature change), pH stimulus (e.g., pH change), or light stimulation (e.g., light irradiation). The property change may be, for example, a property change that facilitates peeling of the culture from the cultureware.

For example, a cell culture is formed by seeding cells on a surface on which a stimulus-responsive polymer is immobilized and culturing the cells in a culture solution in a temperature range where the hydration force of the polymer is weak. After the formation of the cell culture, changing the temperature of the culture solution to a state where the hydration force of the polymer is strong facilitates the peeling of the cell culture from the surface. For example, the temperature range in which the culture is performed (that is, the temperature range in which the hydration force is weak) may be, for example, 33°C to 40°C. The temperature for peeling (that is, the temperature range in which the hydration force is strong) is lower than the above-described temperature range, and may be, for example, 31°C or lower.

In one embodiment of the technology, the stimulus-responsive polymer is a temperature-responsive polymer. When the cells are cultured in a medium containing a cultureware having a surface on which a temperature-responsive polymer is immobilized, for example, after culturing, the temperature of the medium is set to the upper critical dissolution temperature or higher or to the lower critical dissolution temperature or lower of the temperature-responsive polymer, thereby, the surface changes from hydrophobic to hydrophilic, and as a result, peeling between the culture and the porous membrane becomes easier. The culture in the production method of the present invention may be, for example, two-dimensional culture (also called flat culture) or three-dimensional culture (for example, suspension culture, pellet culture, etc.).

When the porous membrane is used in the culturing step, the cells may be in contact with the medium on the upper side of the porous membrane and may be in contact with the medium on the lower side of the porous membrane via the pores of the porous membrane. By culturing in such a state, cells more suitable for cartilage repair can be obtained.

When the cell culture is peeled from the cultureware after the culturing step, treatment with proteolytic enzymes such as dispase and trypsin is unnecessary. By using the properties of the temperature-responsive polymer and changing the temperature of the medium, the cell culture may be peeled from the cultureware. Therefore, the cell culture produced by the production method of the present invention has an advantage that it may be peeled off from the cultureware without being damaged by the enzyme.

Treatment with proteolytic enzymes can result in the degradation of cell-to-cell desmosome structures and cell-to-cultureware basement membrane-like proteins, resulting in the individualization of cells in a cell culture. On the other hand, the cell culture obtained by the production method of the present invention may be peeled off from the cultureware by changing the temperature of the medium without treatment with a proteolytic enzyme. As a result, the desmosome structure is preserved and cell culture defects can be reduced. Moreover, when the cell culture obtained by the production method of the present invention is peeled from the cultureware by changing the temperature of the medium, the basement membrane-like protein is also not destroyed by the enzyme. Therefore, it can adhere better to the diseased tissue at the time of transplantation, and efficient therapy can be performed.

Dispase, which is a proteolytic enzyme, is known to be able to peel the cell sheet while retaining 10 to 60% of the desmosome structure, however, the strength of the resulting cell culture is weak since the enzyme destroys almost the basement membrane-like protein.

The cell culture produced by the production method of the present invention may be peeled off from the cultureware with 80% or more of the desmosome structure and the basement membrane-like protein remaining.

The upper critical dissolution temperature or lower critical dissolution temperature of the temperature-responsive polymer used in the present invention may be preferably 0°C to 80°C, more preferably 20°C to 50°C, and further more preferably 25°C to 45°C. If the upper critical dissolution temperature or lower critical dissolution temperature is too high, cells may die. If the upper critical dissolution temperature or lower critical dissolution temperature is too high, the cell growth rate will slow down or the cells may die.

In the production method of the present invention, the temperature-responsive polymer may be either homopolymer or copolymer. The polymer may be, for example, a homopolymer of a (meth)acrylamide compound, an N-(or N,N-di)alkyl-substituted (meth)acrylamide derivative, or a vinyl ether derivative, or a copolymer of these monomers.

The (meth)acrylamide compound may be, for example, acrylamide or methacrylamide.

Examples of the N-alkyl-substituted (meth)acrylamide derivatives include N-ethylacrylamide (lower critical dissolution temperature of homopolymer: 72°C), N-n-propylacrylamide (the temperature above: 21°C), N-n-propylmethacrylamide (the temperature above: 27°C), N-isopropylacrylamide (the temperature above: 32°C), N-isopropylmethacrylamide (the temperature above: 43°C), N-cyclopropylacrylamide (the temperature above: 45°C), N-cyclopropylmethacrylamide (the temperature above: 60°C), N-ethoxyethyl acrylamide (the temperature above: about 35°C), N-ethoxyethyl methacrylamide (the temperature above: about 45°C), N-tetrahydrofurfuryl acrylamide (the temperature above: about 28°C), or N-tetrahydrofurfuryl methacrylamide (the temperature above: about 35°C).

The N,N-dialkyl-substituted (meth)acrylamide derivative may be, for example, N,N-dimethyl(meth)acrylamide, N,N-ethylmethylacrylamide (lower critical dissolution temperature of homopolymer: 56°C), or N,N-diethylacrylamide (the temperature above: at 32°C).

The vinyl ether derivative may be, for example, methyl vinyl ether (lower critical dissolution temperature of homopolymer 35°C).

In the present invention, a copolymer with a monomer other than the above-mentioned monomers may be used as the temperature-responsive polymer, and further, those obtained by graft polymerizing or copolymerizing polymers, or mixtures of polymers or copolymers may also be used. Further, cross-linking may be carried out within a range that does not impair the original properties of the polymer.

In order to select the temperature-responsive polymer having a critical dissolution temperature more suitable for culturing or peeling in the present invention, to control the interaction between the porous membrane and the culture, or to adjust hydrophilicity or hydrophobicity of the surface of the porous membrane, the above-described polymers can be appropriately selected.

In a preferred embodiment, the above-described temperature responsive polymer is poly(N-isopropylacrylamide).

In the production method of the present invention, the amount of the temperature-responsive polymer immobilized on the surface of the membrane may be preferably 0.3 to 5.0 µg/cm², and more preferably 0.3 to 4.8 µg/cm². In addition, the amount of the temperature-responsive polymer immobilized on the surface of the membrane may be preferably 0.3 and 1.5 µg/cm², when the membrane is a porous membrane, particularly when the membrane is a porous membrane and culturing is performed using a cell culture insert. Further, when the membrane is not a porous membrane, the amount of the temperature-responsive polymer immobilized on the surface of the membrane may preferably be 1 to 2 µg/cm². By setting the immobilized amount of the temperature-responsive polymer within this range, more efficient culturing can be performed than above. If the immobilization amount is outside this range, a cell culture may not be formed or a cell culture may not be produced efficiently. In addition, when the immobilization amount is within this range, the cell culture can be more easily peeled from the membrane.

In the production method of the present invention, it is preferable that the cultureware is a porous membrane, and in the culturing step, the cells are in contact with the medium on the upper side of the porous membrane, and in contact the medium on the lower side of the porous membrane via the pores of the porous membrane. By culturing in such a state, the culturing can be performed more efficiently.

In the production method of the present invention, the material of the membrane, especially the porous membrane, may be for example polycarbonate, polyester, polyethylene terephthalate (PET), polystyrene or polytetrafluoroethylene. Among these, PET is particularly preferred. By using a PET porous membrane, the cell culture of the present invention can be produced more efficiently.

The method for immobilizing the temperature-responsive polymer on the surface of the porous membrane may be carried out, for example, by the method described in JP-A No. hei2-211865. That is, the immobilization may be performed by a method of bonding the porous membrane and the temperature-responsive polymer by a chemical reaction or by a method of bonding by physical interaction. These methods may be used in combination.

In the method of bonding by a chemical reaction, for example, electron beam irradiation (EB), γ-ray irradiation, ultraviolet irradiation, visible light irradiation, LED irradiation, plasma treatment, or corona treatment may be performed. Alternatively, the temperature-responsive polymer may be immobilized on the porous membrane by a generally used organic reaction such as a radical reaction, an anion radical reaction, a cation radical reaction, or the like. Also, a block copolymer having a structure in which a water-insoluble polymer segment and a temperature-responsive polymer segment are bonded may be coated on the surface of a cultureware as the temperature-responsive polymer component. It may be immobilized by physical adsorption or hydrophobic.

In the method of bonding by physical interaction, a temperature-responsive polymer or a mixture of the polymer with any medium may be applied to the porous membrane.

The medium used for culturing in the production method of the present invention may be a medium that can be used for cell culture, particularly mammalian cell culture, such as DMEM/F12 (Dulbecco's Modified Eagle Medium: Nutrient Mixture F-12). The medium may contain additive factors. The additive factors include, for example, cell growth factors, hormones, binding proteins, cell adhesion factors and lipids, and other components.

Examples of the cell growth factors include, but not limited to, TGF-β, b-FGF, IGF, EGF (Epidermal Growth Factor), BMP (Bonemorphogenic protein), Fibroblast growth factor receptor 3 (FGFR-3), Frizzled-related protein (FRZB), CDMP-1, Growth differentiation factor 5 (GDF-5), G-CSF (Granulocyte Colony Stimulating Factor), LIF (Leukemia Inhibitory Factor), interleukin, PDGF (Platelet-Derived Growth Factor), NGF (Nerve Growth Factor), TGF (Transforming Growth Factor) family such as activin A, Wnt family, especially Wnt-3a (Wingless-type MMTV integration site family, member 3A), and the like. The TGF family includes TGF-β1, TGF-β2 and TGF-β3.

The hormones include, but are not limited, insulin, transferrin, dexamethasone, estradiol, prolactin, glucagon, thyroxine, growth hormone, FSH (Follicle Stimulating Hormone), LH (Luteinizing Hormone), glucocorticoids, prostaglandin, and the like.

The above-described cell adhesion factors include, but not limited to, collagen, collagen-like peptides, fibronectin, laminin, and vitronectin. The collagen-like peptides include, for example, recombinant peptides in which RGD sequence-containing regions in collagen are linked. Examples of such recombinant peptides include cellnest (manufactured by FUJIFILM Corporation).

The lipids include, but not limited to, phospholipids, unsaturated fatty acids, and the like.

The other components that can be added to the medium include, but not limited to, ascorbic acid, serum, insulin transferrin selenite (ITS), transferrin, sodium selenite, pyruvic acid, proline, albumin, lipoproteins, ceruloplasmin, and the like. The sera include, but are not limited to, fetal bovine serum (FBS) and human serum. In the production method of the present invention, the medium may be preferably a medium containing FBS, particularly DMEM/F12 containing FBS. The content of FBS is preferably 1 to 30% by volume, preferably 10 to 30% by volume, more preferably 12 to 28% by volume, and further more preferably 15% to 25% by volume with respect to the total volume of the medium for more efficient culturing.

Moreover, in the production method of the present invention, the medium may be a serum-free medium. Since additive factors such as ITS can replace the function of serum, culturing in the production method of the present invention can be performed in a serum-free medium by including the additive factors in the medium. In addition, the use of serum-free media can avoid the risk of using biological raw material resources for transplantation to humans.

It is preferable that the medium used in the present invention may contain ascorbic acid at a content ratio of, for example, 0.01 to 1 mg/mL, preferably 0.05 to 0.5 mg/mL, more preferably 0.07 to 0.3 mg/mL with respect to the medium volume, for better cell growth. Ascorbic acid may enhance the production of joint cartilage-specific substrate from cells in culture and/or render the character expression of a cell culture more suitable for cartilage repair. In other words, ascorbic acid can contribute to regeneration of the damaged site of articular cartilage by hyaline cartilage. If the ascorbic acid concentration is too high, adhesion of cells being cultured to the porous membrane can be prevented. If the ascorbic acid concentration is too low, the effect may not be exhibited.

In a preferred embodiment, the cell culture of the present invention is one obtained by culturing cells in a medium, preferably by culturing cells in a medium containing FBS and/or ascorbic acid, and, for example, may be one obtained by culturing cells in DMEM/F12 containing FBS and/or ascorbic acid.

In the production method of the present invention, the culture period on the membrane of the cells may be appropriately selected depending on the state of the culture, such as the character expression state, and may be, for example, 10 to 20 days, preferably 11 to 18 days, and more preferably 12 to 16 days. Such a culture period may make the cell culture more suitable for cartilage repair.

In the culture, the cell population may be in contact with the medium on the upper side of the porous membrane and in contact with the medium on the lower side of the porous membrane via the pores of the porous membrane. By culturing in this state, adhesion proteins can be produced not only on the surface of the cell sheet adhering to the porous membrane, but also on the surface opposite to the adhesion surface, that is, the surface in contact with the medium on the upper side. The cell sheet of the present invention can be more suitable for cartilage repair by having substrates on both surfaces of the cell sheet.

For culturing in the above-described conditions, for example, culture vessels with cell inserts (also called cell culture inserts) or similar structures may be used. For example, a culture vessel having a cell insert in which a stimulus-responsive polymer (for example, a temperature-responsive polymer) is immobilized on a porous membrane portion may be used in the production method of the present invention. The cell population may be cultured on the surface of the porous membrane on which a stimulus-responsive polymer (for example, temperature-responsive polymer) is immobilized.

Immobilization of the stimulus-responsive polymer may be performed by a method known to those skilled in the art, and for example, may be performed by a method described in JP-A No. hei2-211865. In addition, commercially available cell inserts in which a stimulus-responsive polymer is immobilized may be used, and for example, Thermo Scientific^{™} Nunc^{™} CC insert #140660, BD Falcon cell culture insert #353090, #353490, #353102, #353091, #353092, and #353093 may be used.

In the production method of the present invention, the culture vessel may be appropriately selected by those skilled in the art, and includes, but not limited to, for example, dishes, multiwell plates, flasks, and vessels having shapes similar to them.

The characteristics of the cell culture obtained in the production method of the present invention are as described in 1. above. In addition, the cells cultured in the culturing step and the method for preparing the same are as described in 1. above.

### 2-2. Analysis step

The production method of the present invention preferably includes an analysis step of analyzing the expression intensity of a cell surface marker in the cell population contained in the cell culture. It is preferable that the expression intensity of at least one cell surface marker selected from the group consisting of CD166, CD165, CD99, GD2, STRO-1, CD 108, CD 164, CD6, CD 106, and CD107b, and/or, the expression intensity of at least one cell surface marker selected from the group consisting of CD26, CD73, CD105, CD44, CD120a, CD201, EGFR, CD146, CD140a, and CD90, is measured, in the analysis. The measurement is preferably performed by flow cytometry. The measurement by flow cytometry may be performed as described in "5. Example" below.

In one embodiment of the present invention, the expression intensity of at least CD99 and/or GD2 is measured and/or the expression intensity of at least CD26 and/or CD73 is measured in the analyzing step. These four cell surface markers are particularly suitable for evaluating a cartilage-like tissue forming property, in particular a hyaline cartilage-like forming property.

In another embodiment of the present invention, the expression intensity of at least CD99 and/or GD2 is measured, and/or, the expression intensity of one, two or three of at least CD26, CD73 and CD44 is measured, in the analyzing step. These five cell surface markers are particularly suitable for evaluating a cartilage-like tissue forming property, in particular a hyaline cartilage-like forming property.

In yet another embodiment of the present invention, the expression intensity of at least CD99 and/or GD2 is measured, and/or, the expression intensity of at least CD44 is measured, in the analyzing step. These three cell surface markers are particularly suitable for evaluating a cartilage-like tissue forming property, in particular a hyaline cartilage-like tissue forming property.

### 2-3. Determination step

It is preferable that the production method of the present invention may comprise a determination step of determining whether the cell culture has a cartilage-like tissue forming property (in particular, a hyaline cartilage-like tissue forming property) based on the expression intensity of the cell surface marker obtained in the analysis step.

It may be determined whether the cell culture is suitable for cartilage tissue repair, particularly for hyaline cartilage tissue repair, and more particularly for knee hyaline cartilage tissue repair, in the determination step, based on the expression intensity of the cell surface marker obtained in the analysis step.

It is preferably determined whether the expression intensity of at least one cell surface marker selected from the group consisting of CD166, CD165, CD99, GD2, STRO-1, CD108, CD164, CD6, CD106, and CD107b is not higher than the threshold for each surface marker, and/or, the expression intensity of at least one cell surface marker selected from the group consisting of CD26, CD73, CD105, CD44, CD120a, CD201, EGFR, CD146, CD140a, and CD90 is not lower than the threshold for each surface marker, in the determination step, for the cell population contained in the cell cultured.

It is particularly preferable that when the normalized mean fluorescence intensity measured in analyzing the cell population contained in the cell culture by flow cytometry satisfies at least one of the conditions 1 to 10 described in 1. above, and/or, satisfies at least one of the conditions 11 to 20 described in 1. above, then, it may be determined that the cell culture has a cartilage-like tissue forming property, for example, a hyaline cartilage-like tissue forming property, in the determining step.

In addition, when the normalized mean fluorescence intensity measured in analyzing the cell population contained in the cell culture by flow cytometry satisfies at least one of the conditions 1 to 10 described in 1. above, and/or, satisfies at least one of the conditions 11 to 20 described in 1. above, then, it may be determined that the cell culture is suitable for repairing cartilage tissue, particularly for repairing hyaline cartilage tissue, and more particularly for repairing knee hyaline cartilage tissue, in the determining step.

The cell culture determined to be suitable for cartilage repair in the determining step may be used for transplantation to humans. That is, when the normalized mean fluorescence intensity measured in analyzing the cell population contained in the cell culture by flow cytometry satisfies at least one of the conditions 1 to 10 described in 1. above, and/or, satisfies at least one of the conditions 11 to 20 described in 1. above, then, it may be determined that the cell culture may be used for transplantation to human knee, in the determination step.

### 3. Evaluation method

The present invention also provides a method of evaluating a cell culture. The evaluation method may comprise a determination step of determining whether the cell culture has a cartilage-like tissue forming property (particularly, whether it has a hyaline cartilage-like tissue forming property) based on the expression intensity of the cell surface marker in the cell population contained in the cell culture.

In addition, it may be determined whether the cell culture is suitable for repairing cartilage tissue, particularly for repairing hyaline cartilage tissue, and more particularly for repairing knee hyaline cartilage tissue, based on the expression intensity of the cell surface marker obtained in the analysis step described in 2. above, in the determination step.

This determination step may be the same as the determination step explained in 2. above. For example, the determination may be conducted based on whether the expression intensity, particularly the normalized mean intensity of a specific cell surface marker mentioned in 2. above satisfies the conditions defined for each cell surface maker.

Moreover, the evaluation method may further comprise an analysis step of analyzing the cell population by flow cytometry. The determination in the determination step may be conducted based on the analysis result in the analysis step. This analysis step may be the same as the analysis step explained in 2. above.

The expression intensity of the specific cell surface marker is effective as an index for determining whether a cell culture has a cartilage-like tissue forming property (in particular, whether it has a hyaline cartilage-like tissue forming property). Therefore, the evaluation method of the present invention enables appropriate evaluation of cartilage repair suitability, particularly hyaline cartilage repair suitability.

In addition, in the evaluation method of the present invention, suitability for cartilage repair is determined based on the expression intensity of a cell surface marker, so cartilage repair suitability can be determined without inducing and culturing the cell culture into cartilage-like tissue. Therefore, the time required for cartilage repair suitability can be reduced. Furthermore, since the evaluation method of the present invention can be performed in a short period of time, it can be incorporated as one of the steps in the production of cell culture for cartilage repair, thereby a cell culture having suitability for cartilage repair can be produced more reliably. In addition, the evaluation method of the present invention can also evaluate the cartilage repair suitability at a lower cost than the case of actually inducing and culturing a cell culture into a cartilage-like tissue to evaluate the cartilage repair suitability.

### 4. Marker for evaluating cartilage-like tissue forming property

The present invention provides a marker for evaluating a cartilage-like tissue forming property. The marker comprises at least one cell surface marker selected from the group consisting of CD166, CD165, CD99, GD2, STRO-1, CD108, CD164, CD6, CD 106, and CD107b, and/or, at least one cell surface marker selected from the group consisting of CD26, CD73, CD105, CD44, CD120a, CD201, EGFR, CD146, CD140a, and CD90.

These cell surface markers are suitable for evaluating a cartilage-like tissue forming property of a cell culture.

In one embodiment of the present invention, the marker for evaluating a cartilage-like tissue forming property of the present invention comprises CD99 and/or GD2, and/or, comprises CD26 and/or CD73. These cell surface markers are particularly suitable for evaluating a cartilage-like tissue forming property of a cell culture.

In other embodiments of the present invention, the marker for evaluating a cartilage-like tissue forming property of the present invention comprises CD99 and/or GD2, and/or, comprises one, two, or three of CD26, CD73 and CD44. These cell surface markers are particularly suitable for evaluating a cartilage-like tissue forming property of a cell culture.

In yet another embodiment of the present invention, the marker for evaluating a cartilage-like tissue forming property of the present invention comprises CD99 and/or GD2, and/or, comprises CD44. These cell surface markers are particularly suitable for evaluating a cartilage-like tissue forming property of a cell culture.

Regarding these cell surface markers, for example, a cartilage-like tissue forming property of a cell culture can be evaluated by performing the analysis step and determination step as described in 2. and 3. above.

The present invention also provides a marker for evaluating cartilage repair suitability. This marker includes the cell surface marker described with respect to the marker for evaluating a cartilage-like tissue forming property described above.

### EXAMPLES

The present invention will be described in more detail below based on examples, but the present invention is not limited to these examples.

### 5. Example 1

Cell cultures containing a cell population expressing a specific cell surface marker with specific expression intensities were confirmed to have a cartilage-like tissue forming property, as described below.

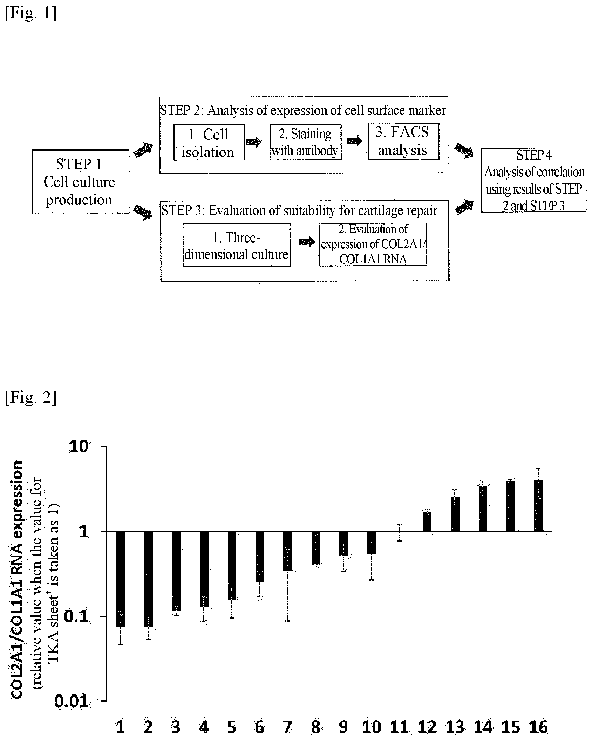

That is, in step 1 of Fig. 1, a plurality of sheet-form cell cultures were prepared. Next, in step 2, expression analysis of a cell surface marker was performed on the plurality of sheet-form cell cultures. For the expression analysis, the sheet-form cell culture was cell-isolated, stained with an antibody, and subjected to FACS analysis. Furter, in step 3, a cartilage-like tissue forming property was evaluated. For the evaluation, three-dimensional culture of the sheet-form cell culture and analysis of the expression level ratio of COL2A1 and COL1A1 were performed. Next, in step 4, correlation analysis was performed using the results of steps 2 and 3. The correlation analysis confirmed that a cell culture containing a cell population expressing a specific cell surface marker with a specific expression intensity has a cartilage-like tissue forming property.

Details of the steps 1 to 4 above are described below.

### 5-1. Step 1: Cell Culture Making

A total of 16 types of cartilage tissue pieces (cartilage derived from tissue discarded during polydactyly surgery) were prepared. Enzyme treatment (Collagenase Type-1/Worthington CLS-1 or Liberase MNP -S/Roche) was applied to each of these cartilage tissue pieces, to isolate chondrocytes from each cartilage tissue piece. The isolated chondrocytes were seeded on a cell culture insert (UpCell (registered trademark) insert, manufactured by CellSeed Inc.). The day of sowing was defined as day 0, and the medium was changed every 3 to 4 days. Ten to twenty-one days after seeding, sheet-form cell cultures derived from chondrocytes in each cartilage tissue piece were obtained.

### 5-2. Step 2: Analysis of expression of cell surface marker

Each of the 16 sheet-form cell cultures obtained in step 1 was washed with PBS and reacted with Accutase (manufactured by Becton Dickinson) at room temperature for 15 minutes. After the reaction, each sheet-form cell culture was collected and Accutase was removed. After the removal, 0.25 mg/ml Collagenase P (Roche) was reacted at 37°C for 30 minutes to disperse the cells in each sheet-form cell culture. The dispersed cells were washed with PBS and suspended in 0.2% FBS/5mM EDTA/PBS to obtain a suspension. Antibodies against each of the 242 types of cell surface markers were added to suspensions containing a cell population obtained from each sheet-form cell culture, and reacted on ice for 30 minutes. Thus, 242 antibody-containing suspensions were obtained for each sheet cell culture. Table 1 below shows antibodies against cell surface markers for which correlation was observed in step 4 below, among these 242 types.

In Table 1 below, "fluorescent pigment" is a fluorescent pigment labeling each antibody. "Target surface antigen" is a cell surface antigen that is targeted by each antibody. "Antibody manufacturer" and "antibody production number" are the manufacturer and production number (catalog number) of each antibody. Regarding the antibody manufacturer, BD is Becton Dickinson, SC is SANTA CRUZ BIOTECHNOLOGY, and BL is BioLegend.

### [Table 1]

**Table 1: List of antibody**

| No. of antibody | Fluorescent pigment | Target surface antigen | Antibody manufacturer | Antibody production number |
|---|---|---|---|---|
| 1 | PE | CD166 | BD | 559263 |
| 2 | BB700 | CD165 | BD | 746053 |
| 3 | FITC | CD99 | BD | 555688 |
| 4 | BB700 | GD2 | BD | 745751 |
| 5 | FITC | STRO-1 | SC | sc47733 |
| 6 | PE | CD108 | BD | 552830 |
| 7 | PE | CD164 | BD | 551298 |
| 8 | FITC | CD6 | BD | 555357 |
| 9 | FITC | CD106 | BD | 551146 |
| 10 | FITC | CD107b | BD | 560946 |
| 11 | FITC | CD26 | BD | 555436 |
| 12 | FITC | CD73 | BD | 561254 |
| 13 | PE | CD105 | BD | 560839 |
| 14 | FITC | CD44 | BD | 555478 |
| 15 | APC | CD120a | BL | 369906 |
| 16 | PE | CD201 | BD | 557950 |
| 17 | PE | EGFR | BD | 555997 |
| 18 | FITC | CD146 | BD | 560846 |
| 19 | PE | CD140a | BD | 556002 |
| 20 | APC | CD90 | BD | 559869 |

After the reaction on ice, each cell population was washed with 0.2% FBS/5 mM EDTA/PBS, and each fluorescence intensity was measured with a flow cytometer (BD FACSVerse, manufactured by Becton Dickinson). Analysis was performed using analysis software Flowjo (available from Becton Dickinson), gated single cells and a live cell population by FSC and SSC, determined the median value of the fluorescence intensity for each fluorescently labeled antibody in the population, and it was normalized by the median value of the fluorescence intensity of unstained samples.

### 5-3. Step 3 (Evaluation of cartilage-like tissue forming property)

A basal medium having the composition shown in Table 2 below was prepared.

### [Table 2]

**Table 2: Composition of basal medium**

| Component | Concentration |
|---|---|
| DMEM/F12 | - |
| FBS | 20 vol% |
| 100×Antibiotic-Antimycotic | 1 vol% |
| 500 mg/5 mL ascorbic acid injection solution | 0.1mg/mL |

The basal medium was supplemented with sodium pyruvate, dexamethasone, L-proline, insulin-transferrin-selenite, and TGF-β1 at the concentrations shown in Table 3 below to obtain an induction medium.

### [Table 3]

**Table 3: Composition of induction medium**

| Component | Concentration |
|---|---|
| Basal medium | - |
| Sodium pyruvate | 1 mM |
| Dexamethasone | 0.1 µM |
| L-proline | 0.35 mM |
| Insulin-transferrin-selenite | 1/500 vol |
| TGF-β1 | 10 ng/mL |

Each sheet-form cell culture was rolled into a sphere and put into a low-adhesion 6-well plate containing the obtained induction medium, and cultured for 7 days under conditions of 5% CO₂ and 37°C. The spherical tissue was collected, RNA was extracted from the spherical tissue, and cDNA was synthesized from the RNA. From the obtained cDNA, the gene expression ratio between COL2A1 and COL1A1 was determined using a qPCR method. Gene expression ratios for each of the 16 sheet-form cell cultures are shown in Fig. 2. In Fig. 2, the gene expression ratio between COL2A1 and COL1A1 in the sheet-form cell culture of donor ID 11 (11 on the horizontal axis) is shown as 1, and the same gene expression ratio in other sheet-form cell cultures is shown as relative values to the gene expression ratios of the sheet-form cell cultures of donor ID 11.

### 5-4. Step 4 (Analysis of correlation)

The above-described gene expression ratios in the sheet-form cell cultures of donor ID 11 are the same as for sheet-form cell cultures made from total artificial joint replacement waste tissue-derived cartilage (hereinafter referred to as "TKA-derived sheet-form cell culture")(Sato M et al., Combined surgery and chondrocyte cell-sheet transplantation improves clinical and structural outcomes in knee osteoarthritis., NPJ Regen Med, 4, 4, 2019.) which have been shown to have a cartilage-like tissue forming property (particularly a hyaline cartilage-like forming property), that is, can be used as a standard for a cell culture having a cartilage tissue forming property. Therefore, the sheet-form cell culture having a COL2A1/COL1A1 gene expression ratio equal to or higher than the COL2A1/COL1A1 gene expression ratio of donor ID 11 can be determined to have a cartilage-like tissue forming property.

242 types of cell surface markers were examined for the presence or absence of correlation with the COL2A1/COL1A1 gene expression ratio by Pearson's product-moment correlation analysis, and it was found that the 20 types of cell surface markers shown in Table 1 had a positive or negative correlation with the COL2A1/COL1A1 gene expression ratio.

Specifically, CD166, CD165, CD99, GD2, STRO-1, CD108, CD164, CD6, CD 106, and CD107b were found to have a negative correlation with the COL2A1/COL1A1 gene expression ratio. Therefore, it can be seen that a cell culture containing a cell population in which the expression intensity of these surface markers is not higher than the threshold can be used as having a cartilage-like tissue forming property and is suitable for cartilage repair.

Further, CD26, CD73, CD105, CD44, CD120a, CD201, EGFR, CD146, CD140a, and CD90 were found to have a positive correlation with the COL2A1/COL1A1 gene expression ratio. Therefore, it can be seen that a cell culture containing a cell population in which the expression intensity of these surface markers is not lower than the threshold can be used as having a cartilage-like tissue forming property and is suitable for cartilage repair.

Furthermore, among the 10 types of cell surface markers that have a negative correlation with the COL2A1/COL1A1 gene expression ratio, CD99 and GD2 were found to have stronger negative correlations (CD99: r = -0.430, GD2: r = -0.397) and have a clear difference from the negative control. Therefore, a cell culture containing a cell population in which expression intensity of at least CD99 and/or GD2 is not higher than the threshold for each of these cell surface markers can be used as having an excellent cartilage-like tissue forming property, and is found to be particularly suitable for cartilage repair (especially hyaline cartilage repair).

In addition, among the 10 types of cell surface markers that have a positive correlation with the COL2A1/COL1A1 gene expression ratio, CD26 and CD73 were found to have stronger positive correlations (CD26: r = 0.485, CD73: r = 0.456) and have a clear difference from the negative control. Therefore, a cell culture containing a cell population in which the expression intensity of at least CD26 and/or CD73 is not lower than the threshold for each of these cell surface markers can be used as having an excellent cartilage-like tissue forming property, and is found to be particularly suitable for cartilage repair (especially hyaline cartilage repair).

Regarding the above 20 types of cell surface markers that have a positive or negative correlation with the COL2A1/COL1A1 gene expression ratio, the nMFI of the sheet-form cell culture with the lowest COL2A1/COL1A1 gene expression ratio, among the sheet-form cell cultures having a COL2A1/COL1A1 gene expression ratio greater than or equal to the COL2A1/COL1A1 gene expression ratio of the TKA-derived sheet-form cell culture, is shown in Table 4 below.

### [Table 4]

**Table 4: Lowest nMFI**

| Cell surface marker with negative correlation | Highest nMFI |
|---|---|
| CD107b | 1.7 |
| CD106 | 2 |
| CD6 | 1.2 |
| CD164 | 2.5 |
| CD108 | 1.6 |
| STRO-1 | 1.2 |
| GD2 | 3 |
| CD99 | 1.6 |
| CD165 | 110 |
| CD166 | 200 |

| Cell surface marker with positive correlation | Lowest nMFI |
|---|---|
| CD26 | 2.9 |
| CD73 | 59 |
| CD105 | 27 |
| CD44 | 160 |
| CD120a | 30 |
| CD201 | 23 |
| EGFR | 4 |
| CD146 | 2 |
| CD140a | 7 |
| CD90 | 1000 |

Regarding the positively correlated cell surface marker CD26, for example, the nMFI of the sheet-form cell culture with the lowest COL2A1/COL1A1 gene expression ratio, among the sheet-form cell cultures with a COL2A1/COL1A1 gene expression ratio greater than or equal to the COL2A1/COL1A1 gene expression ratio of the TKA-derived sheet-form cell culture, is 2.9 as shown in Table 4 above.

Therefore, when a cell population in a sheet-form cell culture is labeled with an FITC-bound anti-CD26 antibody, the normalized mean fluorescence intensity derived from the FITC is, for example, 2.3 or more, more preferably 2.6 or more, and further more preferably 2.9 or more, then, the sheet-form cell culture can be used as having a cartilage-like tissue forming property and is considered to be suitable for cartilage repair.

Similarly, when a cell population in a sheet-form cell culture is labeled with a PE-bound anti-CD166 antibody, the normalized mean fluorescence intensity derived from the PE is, for example, 240 or less, more preferably 220 or less, and further more preferably 200 or less, then, the sheet-form cell culture can be used as having a cartilage-like tissue forming property and is considered to be suitable for cartilage repair.

Similarly, when a cell population in a sheet-form cell culture is labeled with a BB700-bound anti-CD165 antibody, the normalized mean fluorescence intensity derived from the BB700 is 132 or less, more preferably 120 or less, and further more preferably 110 or less, then, the sheet-form cell culture can be used as having a cartilage-like tissue forming property and is considered to be suitable for cartilage repair.

Similarly, when a cell population in a sheet-form cell culture is labeled with an FITC-bound anti-CD99 antibody, the normalized mean fluorescence intensity derived from the FITC is 1.9 or less, more preferably 1.8 or less, and further more preferably 1.6 or less, then, the sheet-form cell culture can be used as having a cartilage-like tissue forming property and is considered to be suitable for cartilage repair.

Similarly, when a cell population in a sheet-form cell culture is labeled with a BB700-bound anti-GD2 antibody, the normalized mean fluorescence intensity derived from the BB700 is 3.6 or less, more preferably 3.3 or less, and further more preferably 3.0 or less, then, the sheet-form cell culture can be used as having a cartilage-like tissue forming property and is considered to be suitable for cartilage repair.

Similarly, when a cell population in a sheet-form cell culture is labeled with an FITC-bound anti-STRO-1 antibody, the normalized mean fluorescence intensity derived from the FITC is 1.4 or less, more preferably 1.3 or less, and further more preferably 1.2 or less, then, the sheet-form cell culture can be used as having a cartilage-like tissue forming property and is considered to be suitable for cartilage repair.

Similarly, when a cell population in a sheet-form cell culture is labeled with a PE-bound anti-CD108 antibody, the normalized mean fluorescence intensity derived from the PE is 1.9 or less, more preferably 1.8 or less, and further more preferably 1.6 or less, then, the sheet-form cell culture can be used as having a cartilage-like tissue forming property and is considered to be suitable for cartilage repair.

Similarly, when a cell population in a sheet-form cell culture is labeled with a PE-bound anti-CD164 antibody, the normalized mean fluorescence intensity derived from the PE is 3.0 or less, more preferably 2.8 or less, and further more preferably 2.5 or less, then, the sheet-form cell culture can be used as having a cartilage-like tissue forming property and is considered to be suitable for cartilage repair.

Similarly, when a cell population in a sheet-form cell culture is labeled with an FITC-bound anti-CD6 antibody, the normalized mean fluorescence intensity derived from the FITC is 1.4 or less, more preferably 1.3 or less, and further more preferably 1.2 or less, then, the sheet-form cell culture can be used as having a cartilage-like tissue forming property and is considered to be suitable for cartilage repair.

Similarly, when a cell population in a sheet-form cell culture is labeled with an FITC-bound anti-CD106 antibody, the normalized mean fluorescence intensity derived from the FITC is 2.4 or less, more preferably 2.2 or less, and further more preferably 2.0 or less, then, the sheet-form cell culture can be used as having a cartilage-like tissue forming property and is considered to be suitable for cartilage repair.

Similarly, when a cell population in a sheet-form cell culture is labeled with an FITC-bound anti-CD107b antibody, the normalized mean fluorescence intensity derived from the FITC is 2.0 or less, more preferably 1.9 or less, and further more preferably 1.7 or less, then, the sheet-form cell culture can be used as having a cartilage-like tissue forming property and is considered to be suitable for cartilage repair.

Similarly, when a cell population in a sheet-form cell culture is labeled with an FITC-bound anti-CD73 antibody, the normalized mean fluorescence intensity derived from the FITC is 47 or more, more preferably 53 or more, and further more preferably 59 or more, then, the sheet-form cell culture can be used as having a cartilage-like tissue forming property and is considered to be suitable for cartilage repair.

Similarly, when a cell population in a sheet-form cell culture is labeled with a PE-bound anti-CD105 antibody, the normalized mean fluorescence intensity derived from the PE is 21 or more, more preferably 24 or more, and further more preferably 27 or more, then, the sheet-form cell culture can be used as having a cartilage-like tissue forming property and is considered to be suitable for cartilage repair.

Similarly, when a cell population in a sheet-form cell culture is labeled with an FITC-bound anti-CD44 antibody, the normalized mean fluorescence intensity derived from the FITC is 128 or more, more preferably 145 or more, and further more preferably 160 or more, then, the sheet-form cell culture can be used as having a cartilage-like tissue forming property and is considered to be suitable for cartilage repair.

Similarly, when a cell population in a sheet-form cell culture is labeled with an APC-bound anti-CD120a antibody, the normalized mean fluorescence intensity derived from the APC is 24 or more, more preferably 27 or more, and further more preferably 30 or more, then, the sheet-form cell culture can be used as having a cartilage-like tissue forming property and is considered to be suitable for cartilage repair.

Similarly, when a cell population in a sheet-form cell culture is labeled with a PE-bound anti-CD201 antibody, the normalized mean fluorescence intensity derived from the PE is 18 or more, more preferably 20 or more, and further more preferably 23 or more, then, the sheet-form cell culture can be used as having a cartilage-like tissue forming property and is considered to be suitable for cartilage repair.

Similarly, when a cell population in a sheet-form cell culture is labeled with a PE-bound anti-EGFR antibody, the normalized mean fluorescence intensity derived from the PE is 3.2 or more, more preferably 3.6 or more, and further more preferably 4.0 or more, then, the sheet-form cell culture can be used as having a cartilage-like tissue forming property and is considered to be suitable for cartilage repair.

Similarly, when a cell population in a sheet-form cell culture is labeled with an FITC-bound anti-CD146 antibody, the normalized mean fluorescence intensity derived from the FITC is 1.6 or more, more preferably 1.8 or more, and further more preferably 2.0 or more, then, the sheet-form cell culture can be used as having a cartilage-like tissue forming property and is considered to be suitable for cartilage repair.

Similarly, when a cell population in a sheet-form cell culture is labeled with a PE-bound anti-CD140a antibody, the normalized mean fluorescence intensity derived from the PE is 5.6 or more, more preferably 6.4 or more, and further more preferably 7.0 or more, then, the sheet-form cell culture can be used as having a cartilage-like tissue forming property and is considered to be suitable for cartilage repair.

Similarly, when a cell population in a sheet-form cell culture is labeled with an APC-bound anti-CD90 antibody, the normalized mean fluorescence intensity derived from the APC is 800 or more, more preferably 900 or more, and further more preferably 1000 or more, then, the sheet-form cell culture can be used as having a cartilage-like tissue forming property and is considered to be suitable for cartilage repair.

### 6. Example 2

Among the cell surface markers mentioned in "5. Example 1" above, CD44 was confirmed to be useful for evaluating a cartilage-like tissue forming property as described below.

That is, in step 2-1, a plurality of sheet-form cell cultures were prepared. Next, in step 2-2, CD44 expression analysis was performed on the plurality of sheet-form cell cultures. In addition, in step 2-3, a plurality of sheet-form cell cultures were three-dimensionally cultured and performed staining treatment to confirm a cartilage-like tissue forming property.

The details of these steps and the results obtained are described below.

### 6-1. Production of cell culture (step 2-1)

A sheet-form cell culture derived from chondrocytes in each cartilage tissue piece was obtained from each of the three types of cartilage tissue pieces by the procedure described in step 1 of "5. Example 1" above. The three sheet-form cell cultures obtained are hereinafter also referred to as "Sample No. 1", "Sample No. 2", and "Sample No. 3", respectively.

### 6-2. Analysis of expression of CD44 (step 2-2)

For each of the three sheet-form cell cultures obtained in step 2-1, the degree of CD44 expression was analyzed by the following cell dispersion treatment and FCM measurement.

### (1) Cell dispersion treatment

### <Reagent>

20% FBS DMEM-F12/AB/AA (hereinafter also referred to as "AA(+) medium")
DPBS (-) (gibco: 14190-250)
Liberase^{™} TL Research Grade (sigma: 54010200001) (hereinafter also referred to as "Liberase TL")
Water for injection (FUJIFILM Wako Pure Chemical Corporation: 161-08247)
5.0 g/l-Trypsin/5.3 mmol/l-EDTA Solution (Nacalai Tesque: 35556-44) (hereinafter also referred to as "Trypsin/EDTA")
0.4% Trypan Blue Stain (Invitrogen, T10282)
STEM-CELLBANKER (ZENOAQ RESOURCE, CB045) (hereinafter also referred to as "cell banker")

### <Procedure>

### <0.65 Units/mL Liberase TL preparation>

1. Add 2 mL of injection water to Liberase TL and mix (hereinafter the resulting mixture is also referred to as "13 Units/mL Liberase TL")
2. Put 5.7 mL of AA(+) medium in a 50 mL tube
3. Add 300 µL of 13 Units/mL Liberase TL and mix (hereinafter the resulting mixture is also referred to as "0.65 Units/mL Liberase TL")

### <Cell dispersion>

1. Prepare an appropriate amount of cell sheets cultured for 14 days
2. Dispense 3 ml/well of DPBS (+) into a 6-well-plate
3. Move a temperature responsive insert (CellSeed: CS6301) to 2.
4. Charge 10 mL of Trypsin/EDTA in a 50 mL tube and warm to 37°C
5. Remove the medium of the insert and wash with DPBS (-) 2 mL/well
6. Peel off the sheet and put it in the tube of 4. (up to 4 sheets/tube)
7. Put in a water bath at 37°C and incubate for 30 min (shake the tube appropriately)
8. Add 10 mL of AA (+) medium to stop enzyme treatment
9. Centrifuge at 350×g for 5 min at 25°C
10. Remove the supernatant
11. Add 3 mL of 0.65 Units/mL Liberase TL
12. Put in a water bath at 37°C and incubate for 30 min (shake the tube appropriately)
   For the following 13. to 16., go to 17. when there are no more cell clumps
13. Add 1 mL of 0.65 Units/mL Liberase TL when cell clumps remain
14. Put in a water bath at 37°C and incubate for 15 min (shake the tube appropriately)
15. Add 1 mL of 0.65 Units/mL Liberase TL when cell clumps remain
16. Put in a water bath at 37°C and incubate for 15 min or longer (shake the tube appropriately, until cell clumps disappear)
17. When cell clumps disappear, add the remaining 0.65 Units/mL Liberase TL
18. Pass through a 40 µm cell strainer and collect in a new 50 mL tube
19. Co-wash the 50 mL tube with 5 mL of AA (+) medium, pass it through the 40 µm cell strainer in 18., and collect it in a 50 mL tube
20. Centrifuge at 350×g for 5 min at 25°C
21. Remove the supernatant
22. Suspend in AA (+) medium
23. Count the number of cells
24. Centrifuge at 350×g for 5 min at 25°C
25. Remove the supernatant
   Perform the following FCM measurement
   If not measure FCM immediately, store it by the following method
26. Suspend in cell banker
27. Dispensing into cryotube
28. Cryopreserve at -80°C
29. After the next day, change to -150°C and store it

### (2) FCM measurement

### <Reagent>

DPBS (-) (gibco: 14190-250)
0.4% Trypan Blue Stain (Invitrogen, T10282)

### <Antibody>

NEG.CTRL IgG1 (mouse)- FITC lOOt CE (Beckman & Coulter: A07795) (hereinafter also referred to as "IgG1-FITC")
MOUSE IgG1-APC, lOOt CE (Beckman & Coulter: IM2475) (hereinafter also referred to as "IgG1-APC")
Hu CD44 FITC G44-26 100Tst (BD Pharmingen: 559596) (hereinafter also referred to as "CD44-FITC")

### <Procedure>

1. Dispense a cell suspension into two tubes at 1×10⁵ to 2×10⁵ viable cells/1.5 mL tube
2. Add 1 mL of PBS (-) and suspend
3. Centrifuge at 350×g for 3 min at 4°C
4. Remove the supernatant
5. Add 1 mL of PBS (-) and suspend
6. Centrifuge at 350×g for 3 min at 4°C
7. Remove the supernatant
8. Add 50 µL of PBS (-) and suspend
9. Add antibody at 5 µL/tube and suspend
   Tube 1: IgG1-FITC●IgG1-APC
   Tube 2: CD44-FITC
10. Shield light and incubate at 4°C for 30 to 60 minutes
11. Add 1 mL of PBS (-) and suspend
12. Centrifuge at 350×g for 3min at 4°C
13. Remove the supernatant
14. Add 0.3 mL of PBS (-) and suspend
15. Add into 5 mL tube with cell strainer
16. Measure and analyze with BD FACSVerse

The measurement and analysis were performed using the BD FACSuite Software Application (available from Becton Dickinson). Specifically, % of CD44 was determined based on 10000 counts and the control (IgG1-FITC) as 1%. Here, "% of CD44" means the proportion of cells stained with an anti-CD44 antibody when the control is taken as 1%. In addition, the range of the histogram in the overlaid state is set so that the control (IgG1-FITC) is 1%, and the numerical value of the count (event) at this time is in the range of 95 to 104.

### 6-3. Three-dimensional culture and staining treatment (step 2-3)

Each of the three kinds of sheet-form cell cultures obtained in step 2-1 was subjected to the following three-dimensional culture treatment and staining treatment.

### (1) Three-dimensional culture treatment

Each sheet-form cell culture was rolled into a sphere and put into a low-adhesion 6-well plate containing the induction medium as described in step 3 of "5. Example 1" above, and cultured under conditions of 5%CO₂ and 37°C for 7 days. After the culture, the spherical tissue was collected.

### (2) Staining treatment

The following safranin O staining treatment was performed on the spherical tissue.

### <Reagent>

Mayer's hematoxylin solution ((FUJIFILM Wako Pure Chemical Corporation, 131-09665)
0.08% Fast Green (prepared reagent)
1% acetic acid water (prepared reagent)
0.1% Safranin O (Muto Pure Chemicals Co., Ltd., 42262)
100% ethanol (FUJIFILM Wako Pure Chemical Corporation, 054-00461)
Xylene (FUJIFILM Wako Pure Chemical Corporation, 241-00096)
Marinol 750cps (Muto Pure Chemicals Co., Ltd., 20091)

### <Procedure for preparing 1% acetic acid>

1. Mix acetic acid and purified water at a ratio of 1:99 in a staining vat

### <Staining procedure>

1. Rinse a glass slide with water for 10 minutes
2. Stain with hematoxylin while checking the staining condition under a microscope (about 10 seconds)
3. Rinse the glass slide with water for 5 minutes
4. Immerse in 0.08% Fast Greens for 5 minutes
5. Immerse in 1% acetic acid water for 10 seconds
6. Immerse in 0.1% Safranin O for 5 minutes
7. Prepare 3 vats containing 100% ethanol and immerse each vat for about 10 seconds while shaking 3 times
8. Prepare 3 vats containing xylene and immerse each vat for about 10 seconds while shaking 3 times
9. Place 2 drops of Marinol on a cover glass and mix with xylene
10. Cover the slide slowly
11. Observe and photograph under a microscope

### 6-4. Results and discussion

The results of expression analysis obtained in step 2-2 above are shown in Table 5 below. The unit in Table 5 below is %. The expression of CD44 in sheet-form cell cultures of Sample No. 2 and Sample No. 3 is stronger than that the expression in Sample No. 1.

### [Table 5]

**Table 5: Result of analysis of expression of CD44**

| Sample No. | CD44 |
|---|---|
| 1 | 83.61 |
| 2 | 94.64 |
| 3 | 94.30 |

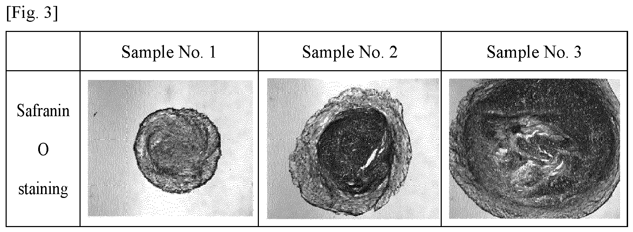

Fig. 3 shows the staining results obtained in step 2-3 above.

As shown in Fig. 3, the spherical tissues of Sample No. 2 and Sample No. 3 are more strongly stained with Safranin O than the spherical tissue of Sample No. 1. A stronger degree of staining with Safranin O in the spherical tissue indicates a superior cartilage-like tissue forming property. Therefore, from the results shown in Fig. 3, the sheet-form cell cultures of Sample No. 2 and Sample No. 3 are superior to Sample No. 1 in the cartilage-like tissue forming property.

From the results shown in Table 3 and the results shown in Fig. 3, it can be seen that the stronger the expression of CD44 in the sheet-form cell culture, the superior cartilage-like tissue forming property. It can also be confirmed that CD44 is useful as an index for evaluating the cartilage-like tissue forming property of a sheet-form cell culture.

In particular, from the viewpoint of the measurement and analysis methods described in "6-2. Analysis of expression of CD44 (step 2-2)" above, it is believed that when % of CD44 is preferably 90% or more, more preferably 92% or more with the control (IgG1-FITC) being 1%, the sheet-form cell culture can be used as a material having a particularly excellent cartilage-like tissue forming property, and is suitable for cartilage repair.

### 6-5. Evaluation based on expression level ratio of COL2 and COL1

It can also be confirmed from the gene expression ratio of COL2 and COL1 and the staining treatment that the cartilage-like tissue forming property of the sheet-form cell culture is excellent when the degree of expression of CD44 in the sheet-form cell culture is high, as described below.

First, from one type of cartilage tissue piece, a sheet-form cell culture derived from chondrocytes in the cartilage tissue piece was obtained, by the procedure as described in step 1 of "5. Example 1" above.

The resulting sheet-form cell culture showed little expression of COL2 and exhibited a high degree of expression of COL1. That is, the sheet-form cell culture was type 2 collagen negative and type 1 collagen positive. Such a sheet-form cell culture has an excellent cartilage-like tissue forming property, as described in International Publication WO2018/154813.

The sheet-form cell culture was subjected to analysis of expression of CD44 as described in step 2-2 above. As a result, the expression of CD44 in the sheet-form cell culture was 94.95, which was comparable to Sample No. 2 and Sample No. 3.

From the above results, it can be confirmed that when the degree of expression of CD44 in the sheet-form cell culture is high, the cartilage-like tissue forming property of the sheet-form cell culture is excellent.

## Claims

1. A cell culture having a cartilage-like tissue forming property, comprising a cell population in which the expression intensity of at least one cell surface marker selected from the group consisting of CD166, CD165, CD99, GD2, STRO-1, CD108, CD164, CD6, CD 106, and CD107b is not higher than the threshold for each cell surface marker, and/or, the expression intensity of at least one cell surface marker selected from the group consisting of CD26, CD73, CD105, CD44, CD120a, CD201, EGFR, CD146, CD140a, and CD90 is not lower than the threshold for each cell surface marker.

2. The cell culture according to claim 1, wherein the expression intensity of at least CD99 and/or GD2 among the cell surface markers is not higher than the threshold for each of these cell surface markers, and/or, the expression intensity of any one, two, or three of at least CD26, CD73, and CD44 among the cell surface markers is not lower than the threshold for each of these cell surface markers.

3. The cell culture according to claim 1 or 2, wherein the expression intensity is a normalized mean fluorescence intensity measured by analyzing the cell population by flow cytometry.

4. The cell culture according to any one of claims 1 to 3, wherein the normalized mean fluorescence intensity measured when analyzing the cell population by flow cytometry satisfies at least one of the following conditions 1 to 10, and/or, satisfies at least one of the following conditions 11 to 20:
Condition 1: when the cell population is labeled with a PE-bound anti-CD166 antibody, the normalized mean fluorescence intensity derived from the PE is 240 or less,
Condition 2: when the cell population is labeled with a BB700-bound anti-CD165 antibody, the normalized mean fluorescence intensity derived from the BB700 is 132 or less,
Condition 3: when the cell population is labeled with an FITC-bound anti-CD99 antibody, the normalized mean fluorescence intensity derived from the FITC is 1.9 or less,
Condition 4: when the cell population is labeled with a BB700-bound anti-GD2 antibody, the normalized mean fluorescence intensity derived from the BB700 is 3.6 or less,
Condition 5: when the cell population is labeled with an FITC-bound anti-STRO-1 antibody, the normalized mean fluorescence intensity derived from the FITC is 1.4 or less,
Condition 6: when the cell population is labeled with a PE-bound anti-CD108 antibody, the normalized mean fluorescence intensity derived from the PE is 1.9 or less,
Condition 7: when the cell population is labeled with a PE-bound anti-CD164 antibody, the normalized mean fluorescence intensity derived from the PE is 3.0 or less,
Condition 8: when the cell population is labeled with an FITC-bound anti-CD6 antibody, the normalized mean fluorescence intensity derived from the FITC is 1.4 or less,
Condition 9: when the cell population is labeled with an FITC-bound anti-CD106 antibody, the normalized mean fluorescence intensity derived from the FITC is 2.4 or less,
Condition 10: when the cell population is labeled with an FITC-bound anti-CD107b antibody, the normalized mean fluorescence intensity derived from the FITC is 2.0 or less,
Condition 11: when the cell population is labeled with an FITC-bound anti-CD26 antibody, the normalized mean fluorescence intensity derived from the FITC is 2.3 or more,
Condition 12: when the cell population is labeled with an FITC-bound anti-CD73 antibody, the normalized mean fluorescence intensity derived from the FITC is 47 or more,
Condition 13: when the cell population is labeled with a PE-bound anti-CD105 antibody, the normalized mean fluorescence intensity derived from the PE is 21 or more,
Condition 14: when the cell population is labeled with an FITC-bound anti-CD44 antibody, the normalized mean fluorescence intensity derived from the FITC is 128 or more,
Condition 15: when the cell population is labeled with an APC-bound anti-CD120a antibody, the normalized mean fluorescence intensity derived from the APC is 24 or more,
Condition 16: when the cell population is labeled with a PE-bound anti-CD201 antibody, the normalized mean fluorescence intensity derived from the PE is 18 or more,
Condition 17: when the cell population is labeled with a PE-bound anti-EGFR antibody, the normalized mean fluorescence intensity derived from the PE is 3.2 or more,
Condition 18: when the cell population is labeled with an FITC-bound anti-CD146 antibody, the normalized mean fluorescence intensity derived from the FITC is 1.6 or more,
Condition 19: when the cell population is labeled with a PE-bound anti-CD140a antibody, the normalized mean fluorescence intensity derived from the PE is 5.6 or more,
Condition 20: when the cell population is labeled with an APC-bound anti-CD90 antibody, the normalized mean fluorescence intensity derived from the APC is 800 or more.

5. The cell culture according to claim 4, wherein the normalized mean fluorescence intensity measured when analyzing the cell population by flow cytometry satisfies the conditions 3 and/or 4, and/or, satisfies one, two or three of the conditions 11, 12, and 14.

6. The cell culture according to any one of claims 1 to 5, wherein the cell culture is in a sheet form.

7. The cell culture according to any one of claims 1 to 6, wherein the cell culture is derived from cartilage tissue.

8. The cell culture according to any one of claims 1 to 7, wherein the cell culture is not derived from synovium.

9. The cell culture according to any one of claims 1 to 6, wherein the cell culture is derived from stem cells.

10. The cell culture according to claim 9, wherein the stem cells include pluripotent stem cells, embryonic stem cells, or somatic stem cells.

11. The cell culture according to any one of claims 1 to 10, wherein the cell culture is used for repairing cartilage tissue.

12. The culture according to any one of claims 1 to 11, wherein the cell culture is used for repairing knee cartilage tissue.

13. The cell culture according to any one of claims 1 to 12, wherein the cell culture has a hyaline cartilage-like tissue forming property.

14. The cell culture according to any one of claims 1 to 13, wherein the cell culture is obtained by culturing cells in a medium containing a cultureware having a surface on which a stimulus-responsive polymer is immobilized.

15. The cell culture according to any one of claims 1 to 13, wherein the cell culture is obtained by culturing cells on the surface of a porous membrane, in which a stimulus-responsive polymer is immobilized on the surface.

16. The cell culture according to any one of claims 1 to 15, wherein the cell culture is obtained by culturing cells in a medium.

17. The cell culture according to any one of claims 1 to 16, wherein the raw material cells used for culturing to obtain the cell culture are cartilage tissue-derived cells, and the cartilage tissue-derived cells are cells obtained by culturing cells in cartilage tissue for at least two days in DMEM/F12 containing FBS.

18. The culture according to any one of claims 1 to 17, wherein the raw material cells used for culturing to obtain the cell culture are prepared by a raw material cell preparation method comprising
a first culturing step of culturing the cells in the cartilage tissue in a medium to make it confluent,
a dissociation step of dissociating the cell population from each other made confluent in the first culturing step, and
a second culturing step of further culturing the cell population dissociated in the dissociation step in the same fresh medium as the medium.

19. A method for evaluating a cell culture, comprising a determination step of determining whether the expression intensity of at least one cell surface marker selected from the group consisting of CD166, CD165, CD99, GD2, STRO-1, CD108, CD164, CD6, CD 106, and CD107b is not higher than the threshold for each surface marker, and/or, the expression intensity of at least one cell surface marker selected from the group consisting of CD26, CD73, CD105, CD44, CD120a, CD201, EGFR, CD146, CD140a, and CD90 is not lower than the threshold for each surface marker, for the cell population contained in the cell culture.

20. The evaluation method according to claim 19, further comprising an analysis step of analyzing the cell population by flow cytometry, wherein the determination in the determination step is performed based on the analysis result in the analysis step.

21. The evaluation method according to claim 19 or 20, wherein, in the determination step, the sheet-form cell culture is determined to have a cartilage-like tissue forming property when the expression intensity of at least one cell surface marker selected from the group consisting of CD166, CD165, CD99, GD2, STRO-1, CD108, CD164, CD6, CD106, and CD107b is not higher than the threshold for each surface marker, and/or, the expression intensity of at least one cell surface marker selected from the group consisting of CD26, CD73, CD105, CD44, CD120a, CD201, EGFR, CD146, CD140a, and CD90 is not lower than the threshold for each cell surface marker.

22. A method for producing a cell culture having a cartilage-like tissue forming property, comprising a culturing step of culturing cells to obtain a cell culture, wherein, in the culturing step, the culturing is performed so that the expression intensity of at least one cell surface marker selected from the group consisting of CD166, CD165, CD99, GD2, STRO-1, CD108, CD164, CD6, CD106, and CD107b is not higher than the threshold for each surface marker, and/or, the expression intensity of at least one cell surface marker selected from the group consisting of CD26, CD73, CD105, CD44, CD120a, CD201, EGFR, CD146, CD140a, and CD90 is not lower than the threshold for each cell surface marker, regarding the cell population contained in the cell culture.

23. The production method according to claim 22, further comprising an analysis step of analyzing the cell population contained in the cell culture by flow cytometry, and
a determination step of determining that the cell culture has a cartilage-like tissue forming property when the expression intensity of at least one cell surface marker selected from the group consisting of CD166, CD165, CD99, GD2, STRO-1, CD108, CD164, CD6, CD106, and CD107b is not higher than the threshold for each surface marker, and/or, the expression intensity of at least one cell surface marker selected from the group consisting of CD26, CD73, CD105, CD44, CD120a, CD201, EGFR, CD146, CD140a, and CD90 is not lower than the threshold for each cell surface marker, in the analysis step.

24. A marker for evaluating a cartilage-like tissue forming property, comprising at least one cell surface marker selected from the group consisting of CD 166, CD165, CD99, GD2, STRO-1, CD 108, CD 164, CD6, CD 106, and CD107b, and/or, at least one cell surface marker selected from the group consisting of CD26, CD73, CD105, CD44, CD120a, CD201, EGFR, CD146, CD140a, and CD90.
